(19) Europäisches Patentamt
European Patent Office
Office européen des brevets

(11) Veröffentlichungsnummer: **0 024 254**
**B1**

(12) # EUROPÄISCHE PATENTSCHRIFT

(45) Veröffentlichungstag der Patentschrift: **10.11.82**

(51) Int. Cl.³: **C 07 D 251/70,** C 07 D 251/64, C 07 D 251/18, C 08 G 59/42

(21) Anmeldenummer: **80810231.3**

(22) Anmeldetag: **21.07.80**

(54) **Aminotriazinpolycarbonsäuren und deren Teilester, deren Herstellung und deren Verwendung als Härter für Epoxidharze.**

(30) Priorität: **25.07.79 GB 7925963**

(43) Veröffentlichungstag der Anmeldung: **25.02.81 Patentblatt 81/8**

(45) Bekanntmachung des Hinweises auf die Patenterteilung: **10.11.82 Patentblatt 82/45**

(84) Benannte Vertragsstaaten: **DE FR GB IT**

(56) Entgegenhaltungen:
**DE-A-1 620 220**
**DE-A-2 121 184**
**US-A-2 763 649**

(73) Patentinhaber: **CIBA-GEIGY AG, Patentabteilung Postfach, CH-4002 Basel (CH)**

(72) Erfinder: **Massy, Derek James Rowland, 1 Green Lane Linton, Cambridge CB1 6JZ (GB)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Aminotriazinpolycarbonsäuren und deren Teilester, deren Herstellung und deren Verwendung als Härter für Epoxidharze

Aus der U.S. Patentschrift Nr. 2 337 220 ist die Umsetzung von Mercaptanen, insbesondere aliphatischen Mercaptocarbonsäuren, mit N-Memethylol- oder N-Halogenmethylderivaten von Amiden bekannt. Die Umsetzung zwischen Thioglykolsäure und einem N-Methylolamid soll dabei zur Bildung eines N-(Carboxymethylthiomethyl)-amids führen, d.h. es findet eine Verätherung und nicht eine Veresterung statt. Die Offenbarungen im einzelnen beschränken sich auf die Verwendung von N-Hydroxymethylamiden aliphatischer Monocarbonsäuren mit mindestens 12 Kohlenstoffatomen, doch sind Amide zahlreicher anderer Arten von Säuren und ferner Harnstoff, Thioharnstoff und Melamin sowie deren Substitutionsprodukte als mögliche Reaktionspartner erwähnt.

Die U.S. Patentschrift Nr. 2 763 649 beschreibt die Bildung von härtbaren, ternären Kondensationsprodukten durch Umsetzung von (a) einem Formaldehydkondensationsprodukt einer Verbindung der Aminotriazin- oder Harnstoffgruppe oder eines Äthers davon mit einem niedermolekularen Alkohol, (b) einer eine Kohlenstoffkette mit mindestens 7 Kohlenstoffatomen und ein an ein Heteroatom gebundenes reaktionsfähiges Wasserstoffatom enthaltenden aliphatischen Verbindung und (c) einer Hydroxy- oder Mercaptocarbonsäure oder Hydroxy- oder Mercaptosulfonsäure. Liegt als (a) ein methyloliertes Aminotriazin oder dessen Äther vor, so setzt man 1 Mol (c) pro Mol (a) ein.

Die Herstellung von Aminotriazinpolycarbonsäuren ist in keiner dieser Literaturstellen offenbart.

Es wurde nun gefunden, dass die Umsetzung eines mindestens zwei N-Methylol- oder N-Alkoxymethylgruppen enthaltenden Aminotriazins mit mindestens zwei Äquivalenten einer Mercaptocarbonsäure Aminotriazinpolycarbonsäuren ergibt, worin die Carboxylgruppen über Thioätherbrücken an den Aminotriazinring gebunden sind. Wenn man ferner von einem Poly-(alkoxymethylamino)-triazin ausgeht, kann in gewissem Umfang eine nachfolgende Veresterung stattfinden, so dass das Produkt nicht nur die Aminotriazinpolycarbonsäure sondern auch deren Teilester enthalten kann, wobei die veresternde Gruppe die gleiche ist wie die ursprüngliche veräthernde Gruppe; mit Methoxymethylaminotriazinen können sich also ebenfalls Methylester der Polycarbonsäuren bilden. Es wurde ferner gefunden, dass man durch Ersatz eines Anteils der Mercaptocarbonsäure durch ihren Ester oder durch teilweise Umsetzung der Aminotriazinpolycarbonsäure mit einem Alkohol Teilester erhalten kann.

Polycarbonsäuren sind wohlbekannt als Härter für Epoxidharze, d.h. Stoffe, die mehr als eine 1,2-Epoxidgruppe pro Durchschnittsmolekül enthalten. Solche bekannten Säuren erfordern häufig eine hohe Temperatur und/oder längeres Erhitzen, um das Harz auszuhärten. Es wurde ferner gefunden, dass die neuen Polycarbonsäuren und deren mindestens zwei Carboxylgruppen enthaltende Teilester Epoxidharze bei nur kurzzeitigem Erhitzen auf mässige Temperaturen härten.

Gegenstand vorliegender Erfindung sind demnach Aminotriazinpolycarbonsäuren und deren Teilester mit mindestens zwei freien Carboxylgruppen, der allgemeinen Formel

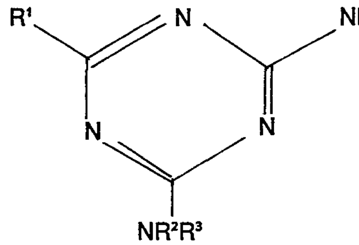

I

worin $R^1$ für eine Gruppe der Formel $-NR^2R^3$, eine geradkettige oder verzweigte Alkylgruppe mit 1 bis 17 Kohlenstoffatomen, eine einkernige Arylgruppe mit 6 bis 10 Kohlenstoffatomen, eine Phenylalkylengruppe mit 1 bis 4 Kohlenstoffatomen im Alkylenteil oder eine Gruppe der Formel

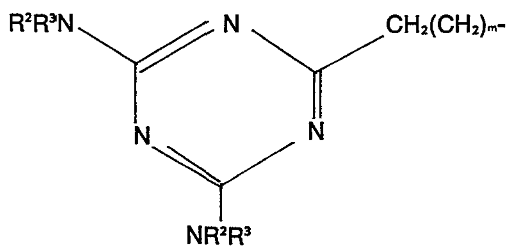

II

steht, $R^2$ und $R^3$ gleich oder verschieden sein können und je ein Wasserstoffatom, eine Gruppe der Formel $-CHR^4OR^5$, eine Gruppe der Formel $-CHR^4-S-R^6-COOQ$ oder eine Gruppe der Formel

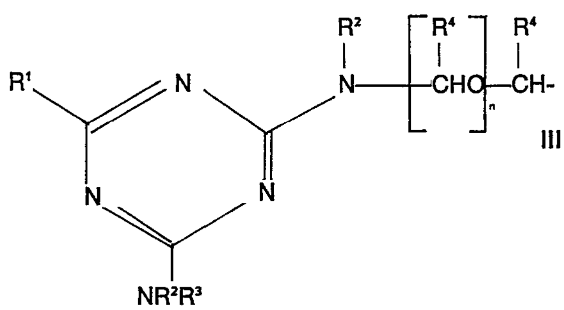

III

darstellen, wobei $R^2$ und $R^3$ in den Formeln I und III so ausgewählt sind, dass höchstens eine davon eine Gruppe der Formel III und mindestens zwei davon eine Gruppe der Formel $-CHR^4-S-R^6-COOQ$ bedeuten, m eine ganze Zahl von 1 bis 3 ist, $R^4$ jeweils für ein Wasserstoffatom oder eine geradkettige oder verzweigte Alkylgruppe mit 1 bis 3 Kohlenstoffatomen, $R^5$ jeweils für ein Wasserstoffatom oder eine geradkettige oder verzweigte Alkylgruppe mit 1 bis 8

und vorzugsweise 1 bis 4 Kohlenstoffatomen und $R^6$ jeweils für eine gegebenenfalls durch eine weitere Gruppe -COOQ substituierte, zweiwertige geradkettige oder verzweigte Alkylengruppe mit 1 bis 4 Kohlenstoffatomen stehen, Q jeweils ein Wasserstoffatom oder eine geradkettige oder verzweigte Alkylgruppe mit 1 bis 8 Kohlenstoffatomen darstellt, derart, dass im Durchschnitt jeweils mindestens zwei Q in der Formel I für ein Wasserstoffatom stehen, und n null oder 1 ist.

Wie oben angedeutet kann eines (aber nicht mehr als eines) der verschiedenen Symbole $R^2$ und $R^3$ in der Formel I einen Triazinring der Formel III darstellen. Ein Symbol $R^2$ oder $R^3$ in diesem Ring kann noch einen weiteren Ring der Formel III darstellen, und auf diese Weise lässt sich eine Kette von substituierten, durch Alkylen- oder Alkylenätherbrücken verknüpften Triazinringen bilden. Um die Herstellung der neuen Verbindungen zu erleichtern und bei der Härtung von Epoxidharzen die besten Ergebnisse zu erzielen, weisen die Säuren und Ester der Formel I vorzugsweise ein Molekulargewicht von höchstens 2000 auf.

Die Gruppe $R^1$ ist vorzugsweise eine Gruppe $-NR^2R^3$, wobei $R^2$ und $R^3$ die oben angegebene Bedeutung haben, aber sie kann auch beispielsweise eine Heptadecyl-, Nonyl-, Propyl- oder Benzylgruppe und insbesondere eine Methyl-, Phenyl- oder Undecylgruppe sein.

Vorzugsweise stellen alle Symbole $R^4$ je ein Wasserstoffatom dar. Mindestens ein Q und mindestens ein $R^5$ stehen vorzugsweise für eine Alkylgruppe, wie eine Methyl-, n-Butyl-, Isobutyl- oder 2-Äthylhexylgruppe. Alle Gruppen $R^6$ in der Formel I bedeuten vorzugsweise eine Methylen-, Äthylen- oder 2-Carboxyäthylidengruppe.

Weitere bevorzugte Substanzen der Formel I sind die Säuren und Ester, die im wesentlichen auch der Formel

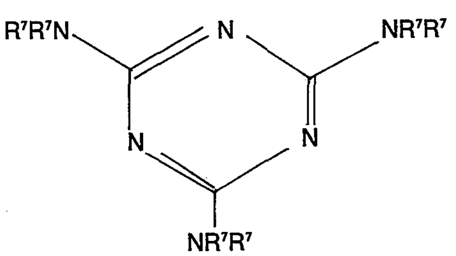

IV

worin zwei bis sechs von den $R^7$ je für eine Gruppe der Formel $-CH_2SCH_2COOQ$, $-CH_2SCH_2CH_2COOQ$, $-CH_2SCH(COOQ)CH_2COOQ$ oder $-CH_2SCH(CH_3)COOQ$, wobei Q die oben angegebene Bedeutung hat und gegebenenfalls verbleibende $R^7$ je ein Wasserstoffatom bedeuten, entsprechen, sowie solche, die im wesentlichen auch der Formel

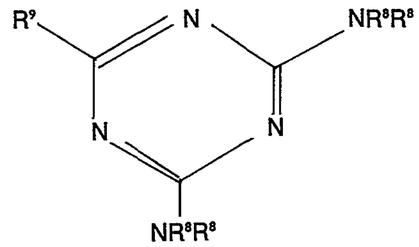

V

worin zwei bis vier von den $R^8$ je für eine Gruppe der Formel $-CH_2SCH_2COOQ$, $-CH_2SCH_2COOQ$, $-CH_2SCH(COOQ)CH_2COOQ$ oder $-CH_2SCH(CH_3)-COOQ$ stehen, wobei Q die oben angegebene Bedeutung hat und gegebenenfalls verbleibende $R^8$ je ein Wasserstoffatom bedeuten, und $R^9$ eine Methyl-, Phenyl- oder Undecylgruppe darstellt, entsprechen.

Spezielle Beispiele für Säuren der Formel I sind Hexakis-(N-2-carboxyäthylthiomethyl)-melamin, Hexakis-(N-carboxymethylthiomethyl)-melamin, Tetrakis-(N-2-carboxymethylthiomethyl)-acetoguanamin, Tetrakis-(N-2-carboxyäthylthiomethyl)-benzoguanamin und Tetrakis-(N-2-carboxyäthylthiomethyl)-lauroguanamin.

Die Säuren und Teilester der Formel I lassen sich durch Umsetzung von mindestens 2 Moläquivalenten einer Mercaptocarbonsäure der allgemeinen Formel

$$HS-R^6-COOH \qquad VI$$

mit einem Moläquivalent eines gegebenenfalls alkylierten Kondensationsprodukts aus einem Aminotriazin und einem Aldehyd, der allgemeinen Formel

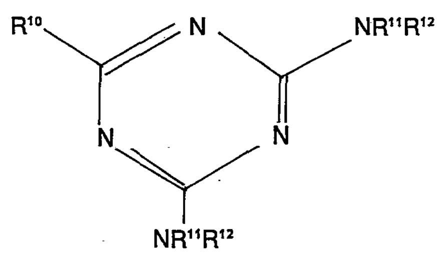

VII

worin $R^6$ die oben angegebene Bedeutung hat, $R^{10}$ eine Gruppe $-NR^{11}R^{12}$ oder eine geradkettige oder verzweigte Alkylgruppe mit 1 bis 17 Kohlenstoffatomen, eine einkernige Arylgruppe mit 6 bis 10 Kohlenstoffatomen, eine Phenylalkylengruppe mit 1 bis 4 Kohlenstoffatomen im Alkylenteil oder eine Gruppe der Formel

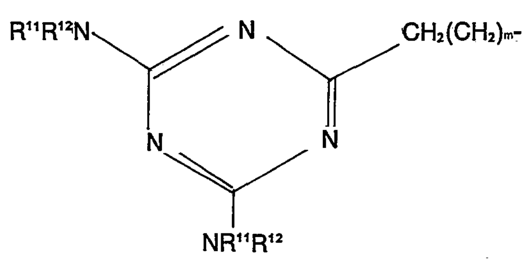

VIII

darstellt, $R^{11}$ und $R^{12}$ gleich oder verschieden sein können und je ein Wasserstoffatom, eine Gruppe $-CH^4-O-R^5$ oder eine Gruppe der Formel

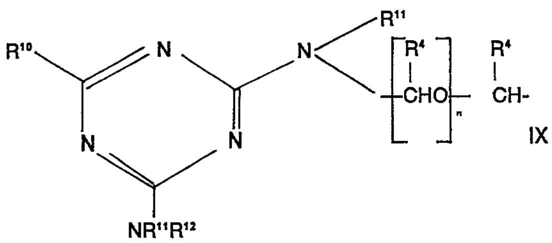

IX

bedeuten, wobei $R^4$, $R^5$, m und n die oben angegebene Bedeutung haben und $R^{11}$ und $R^{12}$ in der Verbindung der Formel VII so ausgewählt sind, dass höchstens eines davon eine Gruppe der Formel IX und mindestens zwei davon eine Gruppe der Formel $-CHR^4-OR^5$ bedeuten, herstellen.

Diese Umsetzung lässt sich so durchführen, dass man die Komponenten bei Raumtemperatur oder vorzugsweise erhöhter Temperatur, üblicherweise zwischen 50° und 150°C, entweder ohne Lösungsmittel oder in Gegenwart eines geeigneten Lösungsmittels wie Wasser, Methanol, Äthanol, Dioxan, Aceton, Eisessig, Benzol oder Toluol und gewünschtenfalls in Gegenwart eines sauren Katalysators wie Schwefelsäure oder Salzsäure vermischt.

Wenn mindestens eine der Gruppen $R^{11}$ und $R^{12}$ in der Formel VII eine Gruppe der Formel $-CHR^4-O-R^5$ darstellt, wobei $R^5$ für eine Alkylgruppe wie oben angegeben steht, ist das Produkt üblicherweise ein Gemisch aus der Säure der Formel I, worin Q für ein Wasserstoffatom steht, und einer untergeordneten Menge des Esters der Formel I, worin Q für eine gleichartige Alkylgruppe $R^5$, vorzugsweise eine Methyl-, n-Butyl-, Isobutyl- oder 2-Äthylhexylgruppe steht. Falls erwünscht, lassen sich solche Gemische nach herkömmlichen Methoden trennen, doch sind sie üblicherweise ohne eine solche Trennung als Härter für Epoxidharze einsetzbar. Die Anwendung der Teilester ist häufig vorteilhaft, beispielsweise wenn das gehärtete Epoxidharz biegsamer sein soll als man mit ausschliesslich einer unveresterten erfindungsgemässen Polycarbonsäure als Härter erhalten würde.

Nach einer Abänderung des obigen erfindungsgemässen Verfahrens wird ein Ester der Formel

$$HS - R^6 - COOQ^1 \qquad X$$

worin $R^6$ die oben angegebene Bedeutung hat und $Q^1$ eine geradkettige oder verzweigte Alkylgruppe mit 1 bis 8 Kohlenstoffatomen, z.B. eine Methyl-, n-Butyl-, Isobutyl- oder 2-Äthylhexylgruppe bedeutet, als einer der Reaktionspartner mitverwendet, um einen Teilester der Formel I zu erhalten.

Diese Abänderung lässt sich anstelle der Verwendung eines alkylierten Aminotriazin/Aldehydkondensationsprodukts (d.h. einer Verbindung der Formel V, worin mindestens eine der Gruppen $R^{11}$ und $R^{12}$ eine Gruppe der Formel $-CHR^4-O-R^5$ darstellt, wobei $R^5$ für eine Alkylgruppe wie oben angegeben steht) anwenden, oder auch bei Einsatz eines solchen alkylierten Kondensationsproduktes, um den Gehalt an Estergruppen zu erhöhen (die gleich oder verschieden von den durch Umsetzung mit dem freigesetzten Alkohol gebildeten Estergruppen sein können).

Bei einer weiteren Herstellungsmethode für die erfindungsgemässen Teilester werden die Aminotriazinpolycarbonsäuren der Formel I, worin mindestens drei Q je ein Wasserstoffatom darstellen, auf herkömmliche Weise unter Verwendung eines Alkohols der Formel

$$Q^1OH \qquad XI$$

worin $Q^1$ die oben angegebene Bedeutung hat, verestert.

Der Anteil eines Esters der Formel X oder eines Alkohols der Formel XI sollte natürlich nicht so hoch liegen, dass das Produkt durchschnittlich weniger als zwei freie Carboxylgruppen pro Molekül enthält, da das Produkt sonst als Härter für Epoxidharze unbrauchbar wäre.

Eine andere Herstellungsmethode für Säuren und Teilester der Formel I besteht darin, dass man mindestens zwei Moläquivalente einer Mercaptocarbonsäure der Formel VI und gegebenenfalls eine entsprechende Menge eines Esters der Formel X mit einem Moläquivalent eines Aminotriazins der Formel

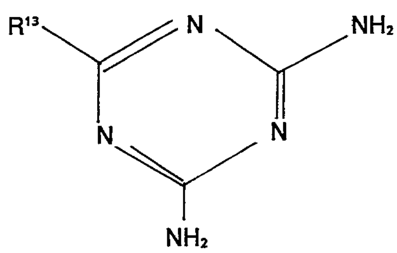

XII

und mindestens zwei Moläquivalente eines Aldehyds der Formel

$$R^4CHO \qquad XIII$$

worin $R^{13}$ eine unsubstituierte Aminogruppe, eine geradkettige oder verzweigte Alkylgruppe mit 1 bis 17 Kohlenstoffatomen, eine einkernige Arylgruppe mit 6 bis 10 Kohlenstoffatomen, eine Phenylalkylengruppe mit 1 bis 4 Kohlenstoffatomen im Alkylenteil oder eine Gruppe der Formel

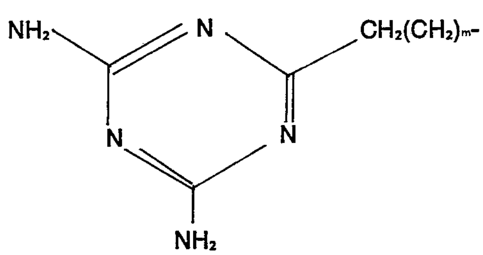

XIV

darstellt sowie m und $R^4$ die oben angegebene Bedeutung haben, umsetzt.

Vorzugsweise erfolgt diese Umsetzung unter wässrigen Bedingungen, wobei man die Säure

der Formel IV, gegebenenfalls den Ester der Formel X sowie das Aminotriazin der Formel XII gegebenenfalls unter Erhitzen in Berührung hält, bis sie sich infolge Salzbildung auflösen, und dann den Aldehyd der Formel XIII zusetzt. Das Gemisch wird dann weitergerührt, bis die Umsetzung praktisch vollständig ist. Die Umsetzung kann bei Raumtemperatur erfolgen, doch erhitzt man die Reaktionspartner vorzugsweise auf eine Temperatur von 50° bis 100°C.

Bei einer Variante der obigen Methode unter Einsatz derselben Molanteile kann man die Säure der Formel VI (und gegebenenfalls den Ester der Formel X) zunächst unter wässrigen oder wasserfreien Bedingungen mit dem Aldehyd der Formel XIII vermischen und danach das Aminotriazin der Formel XII zusetzen. Diese Umsetzung kann ebenfalls bei Raumtemperatur oder erhöhter Temperatur erfolgen, und vorzugsweise erhitzt man die Reaktionspartner auf eine Temperatur von 50° bis 100°C.

Eine besonders bevorzugte Ausführungsform dieser Erfindung besteht darin, dass man Melamin in Abwesenheit eines Esters der Formel X mit 2 bis 6 Moläquivalenten Thioglykolsäure, 2-Mercaptopropionsäure, 3-Mercaptopropionsäure oder Mercaptobernsteinsäure und mit 2 bis 6 Moläquivalenten Formaldehyd umsetzt, wobei die gleiche Anzahl Äquivalente des Formaldehyds und der Mercaptosäure angewendet werden. Bei einer weiteren besonders bevorzugten Ausführungsform setzt man Acetoguanamin, Benzoguanamin oder Lauroguanamin in Abwesenheit eines Esters der Formel X mit 2 bis 4 Moläquivalenten Thioglykolsäure, 2-Mercaptopropionsäure, 3-Mercaptopropionsäure oder Mercaptobernsteinsäure um, wobei die gleiche Anzahl Äquivalente des Formaldehyds und der Mercaptosäure angewendet werden.

Aminotriazinpolycarbonsäuren der Formel I, worin sämtliche $R^4$ und Q je für ein Wasserstoffatom stehen, lassen sich auch durch Umsetzung eines Moläquivalents eines Aminotriazins der allgemeinen Formel

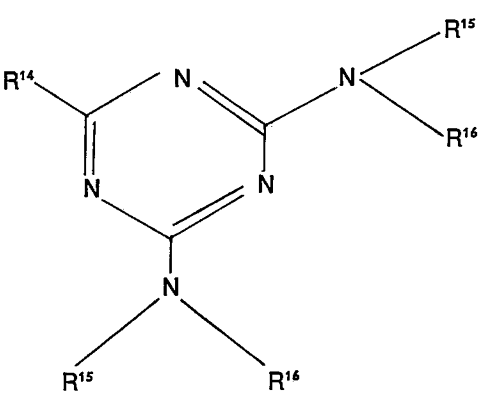

XV

mit mindestens zwei Moläquivalenten einer Mercaptocarbonsäure der obigen Formel VI herstellen, wobei $R^{14}$ eine Gruppe der Formel $-NR^{15}R^{16}$, eine geradkettige oder verzweigte Alkylgruppe mit 1 bis 17 Kohlenstoffatomen, eine einkernige Arylgruppe mit 6 bis 10 Kohlenstoffatomen, eine Phenylalkylengruppe mit 1 bis 4 Kohlenstoffatomen im Alkylenteil oder eine Gruppe der Formel

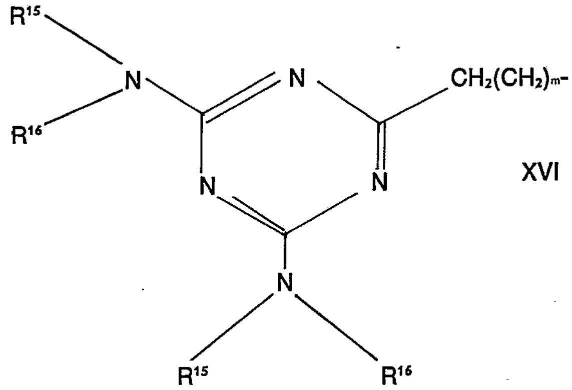

XVI

bedeutet, worin m die oben angegebene Bedeutung hat, $R^{15}$ und $R^{16}$ gleich oder verschieden sein können und je für ein Wasserstoffatom oder eine Gruppe der Formel $-CH_2NR^{17}R^{18}$ stehen, worin entweder $R^{17}$ ein Wasserstoffatom oder eine geradkettige oder verzweigte Alkylgruppe mit 1 bis 4 Kohlenstoffatomen und $R^{18}$ eine solche (gleiche oder verschiedene) Alkylgruppe darstellen oder $R^{17}$ und $R^{18}$ zusammen mit dem Stickstoffatom, an das sie gebunden sind, einen einwertigen, einkernigen heterocyclischen Rest mit bis zu 8 Kohlenstoffatomen bedeuten, wobei $R^{15}$ und $R^{16}$ so ausgewählt werden, dass die Verbindung der Formel XV mindestens zwei Gruppen der Formel $-CH_2NR^{17}R^{18}$ aufweist, und wobei die Umsetzung dadurch erfolgt, dass man die Reaktionspartner miteinander entweder allein oder in einem Lösungsmittel wie Xylol, Methanol, Äthanol, Dioxan, Aceton, Eisessig, Benzol, Toluol oder Pyridin unter Abspaltung des Amins der Formel

$$R^{17}-NH-R^{18} \qquad XVII$$

erhitzt.

Üblicherweise erhitzt man die Reaktionspartner auf eine Temperatur im Bereich von 100° bis 200°C.

Die als Ausgangsstoffe verwendeten Aminotriazine der Formel XV lassen sich durch eine Mannichreaktion zwischen einem Aminotriazin der Formel XII, Formaldehyd und einem primären oder sekundären Amin der Formel XVII oder einem Salz eines solchen Amins herstellen. Die Art des eingesetzten Amins ist im allgemeinen nicht kritisch, doch wird es aus Gründen der Sparsamkeit und der Vermeidung unerwünschter Nebenprodukte und zur Erleichterung der Abspaltung während der Umsetzung mit der Mercaptocarbonsäure bevorzugt, dass $R^{17}$ für ein Wasserstoffatom und $R^{18}$ für eine Methyl- oder Äthylgruppe oder sowohl $R^{17}$ als auch $R^{18}$ für eine Methyl- oder Äthylgruppe stehen oder $R^{17}$ und $R^{18}$ zusammen eine Gruppe der Formel $-(CH_2)_5-$ oder eine Gruppe der Formel $-(CH_2)_2O-(CH_2)_2-$ darstellen.

Wie oben angegeben sind die erfindungsgemässen Säuren und Teilester als Härter für Epoxidharze nützlich.

Weitere Gegenstände der vorliegenden Erfindung sind dementsprechend härtbare Zusammensetzungen, die ein Epoxidharz und eine Aminotriazinpolycarbonsäure oder einen Teilester der Formel I enthalten, sowie ein Verfahren zum Härten eines Epoxidharzes, welches darin besteht, dass man eine solche Zusammensetzung herstellt und erhitzt.

Dabei wird eine wirksame, d.h. härtende Menge der Polycarbonsäure oder des Esters eingesetzt. Das Verhältnis zwischen der Säure oder dem Teilester und dem Epoxidharz hängt dabei von der eingesetzten jeweiligen Säure bzw. Ester und von den für die härtbare Zusammensetzung und das gehärtete Produkt daraus erwünschten Eigenschaften ab. Das optimale Verhältnis lässt sich leicht nach dem Fachmann vertrauten Methoden bestimmen. Als Erläuterung sei jedoch angegeben, dass man normalerweise so viel Polycarbonsäure oder Teilester einsetzt, dass sich 0,8 bis 1,2 Carboxylgruppen pro Epoxidgruppe im Epoxidharz ergeben.

In diesen Zusammensetzungen verwendbare Epoxidharze sind vorzugsweise solche, welche direkt an Sauerstoff-, Stickstoff- oder Schwefelatome gebundene Gruppen der Formel

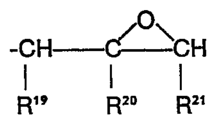

XVIII

enthalten, worin entweder $R^{19}$ und $R^{21}$ je ein Wasserstoffatom darstellen, in welchem Fall $R^{20}$ ein Wasserstoffatom oder eine Methylgruppe bedeutet, oder $R^{19}$ und $R^{21}$ zusammen $-CH_2CH_2-$ darstellen, in welchem Fall $R^{20}$ ein Wasserstoffatom bedeutet.

Als Beispiele solcher Harze seien Polyglycidyl- und Poly-(β-methylglycidyl)-ester genannt, die man durch Umsetzung einer zwei oder mehr Carbonsäuregruppen pro Molekül enthaltenden Verbindung mit Epichlorhydrin, Glycerindichlorhydrin oder β-Methylepichlorhydrin in Gegenwart von Alkali erhalten kann. Solche Polyglycidylester können sich von aliphatischen Polycarbonsäuren, z.B. Oxalsäure, Bernsteinsäure, Glutarsäure, Adipinsäure, Pimelinsäure, Korksäure, Azelainsäure, Sebacinsäure oder dimerisierter oder trimerisierter Linolsäure; von cycloaliphatischen Polycarbonsäuren wie Tetrahydrophthalsäure, 4-Methyltetrahydrophthalsäure, Hexahydrophthalsäure und 4-Methylhexahydrophthalsäure; und von aromatischen Polycarbonsäuren wie Phthalsäure, Isophthalsäure und Terephthalsäure ableiten.

Weitere Bespiele sind Polyglycidyl- und Poly-(β-methylglycidyl)-äther, die durch Umsetzung einer mindestens zwei freie alkoholische und/oder phenolische Hydroxylgruppen pro Molekül enthaltenden Verbindung mit dem entsprechenden Epichlorhydrin unter alkalischen Bedingungen, oder auch in Gegenwart eines sauren Katalysators mit nachfolgender Alkalibehandlung, erhältlich sind. Diese Äther lassen sich mit Poly-(epichlorhydrin) aus acyclischen Alkoholen wie Äthylenglykol, Diäthylenglykol und höheren Poly-(oxyäthylen)-glykolen, Propan-1,2-diol und Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Pentaerythrit und Sorbit; aus cycloaliphatischen Alkoholen wie Resorcit, Chinit, Bis-(4-hydroxycyclohexyl)-methan, 2,2-Bis-(4-hydroxycyclohexyl)-propan und 1,1-Bis-(hydroxymethyl)-cyclohexen-3; und aus Alkoholen mit aromatischen Kernen wie N,N-Bis-(2-hydroxyäthyl)-anilin und p,p'-Bis-(2-hydroxyäthylamino)-diphenylmethan herstellen. Man kann sie ferner aus einkernigen Phenolen wie Resorcin u. Hydrochinon u. mehrkernigen Phenolen wie Bis-(4-hydroxyphenyl)-methan, 4,4'-Dihydroxydiphenyl, Bis-(4-hydroxyphenyl)-sulfon, 1,1,2,2-Tetrakis-(4-hydroxyphenyl)-äthan, 2,2-Bis-(4-hydroxyphenyl)-propan (sonst als Bisphenol A bekannt) und 2,2-Bis-(3,5-dibrom-4-hydroxyphenyl)-propan sowie aus Aldehyden wie Formaldehyd, Acetaldehyd, Chloral und Furfurol mit Phenolen wie Phenol selbst und durch Chloratome oder Alkylgruppen mit jeweils bis zu neun Kohlenstoffatomen ringsubstituiertem Phenol wie 4-Chlorphenol, 2-Methylphenol und 4-tert.Butylphenol gebildeten Novolaken herstellen.

Poly-(N-glycidyl)-Verbindungen umfassen beispielsweise solche, die durch Dehydrochlorierung der Umsetzungsprodukte von Epichlorhydrin mit mindestens zwei Aminowasserstoffatome enthaltenden Aminen wie Anilin, n-Butylamin, Bis-(4-aminophenyl)-methan und Bis-(4-methylaminophenyl)-methan erhalten werden; Triglycidylisocyanurat; sowie N,N'-Diglycidylderivate von cyclischen Alkylenharnstoffen wie Äthylenharnstoff und 1,3-Propylenharnstoff, und Hydantoinen wie 5,5-Dimethylhydantoin.

Poly-(S-glycidyl)-Verbindungen sind zum Beispiel die Di-S-glycidylderivate von Dithiolen wie Äthan-1,2-dithiol und Bis-(4-mercaptomethylphenyl)-äther.

Beispiele für Epoxidharze mit Gruppen der Formel XVIII, worin $R^{19}$ und $R^{21}$ zusammen eine $-CH_2CH_2-$Gruppe bedeuten, sind Bis-(2,3-epoxycyclopentyl)-äther, 2,3-Epoxycyclopentyl-glycidyläther und 1,2-Bis-(2,3-epoxycyclopentyloxy)-äthan.

In Betracht kommen auch Epoxidharze, in denen die 1,2-Epoxidgruppen an Heteroatome verschiedener Art gebunden sind, beispielsweise das N,N,O-Triglycidylderivat des 4-Aminophenols, der Glycidyläther/Glycidylester der Salicylsäure, N-Glycidyl-N'-(2-glycidyloxypropyl)-5,5-dimethylhydantoin und 2-Glycidyloxy-1,3-bis-(5,5-dimethyl-1-glycidylhydantoinyl-3)-propan.

Ebenfalls einsetzbar sind auch Epoxidharze, in denen einige oder sämtliche Epoxidgruppen mittelständig sind, wie Vinylcyclohexendioxyd, Limonendioxyd, Dicyclopentadiendioxyd, 4-Oxatetracyclo[6.2.1.$0^{2,7}$.$0^{3,5}$]undecyl-(9)-glycidyläther, 1,2-Bis-(4-oxatetracyclo[6.2.1.$0^{2,7}$.$0^{3,5}$]-undecyl--(9)-oxy)-äthan, der 3,4-Epoxycyclohexylmethylester der 3',4'-Epoxycyclohexancarbonsäure sowie dessen 6,6'-Dimethylderivat, der Bis-(3,4--epoxycyclohexancarbonsäureester) des Äthylenglykols, Adipinsäure-bis-(3,4-epoxy-6-methylcyclohexylester), 3-(3,4-Epoxycyclohexyl)-8,9-epoxy-2,4-dioxaspiro[5,5]undecan sowie epoxidiertes Butadien bzw. Copolymere des Butadiens mit Äthylenverbindungen z.B. Styrol und Acrylnitril.

Gewünschtenfalls kann man Epoxidharzgemische verwenden.

Bevorzugte Epoxidharze sind die Polyglycidyläther, Polyglycidylester und N,N'-Diglycidylhydantoine, insbesondere solche, die einen 1,2--Epoxidgehalt von mehr als 0,5 Äquivalent pro Kilogramm aufweisen. Spezielle bevorzugte Harze sind N,N'-Diglycidyl-5,5-dimethylhydantoin und die Polyglycidyläther von 2,2-Bis-(4-hydroxyphenyl)-propan, von Bis-(4-hydroxyphenyl)-methan oder aus Formaldehyd und Phenol oder durch ein Chloratom oder eine Alkylkohlenwasserstoffgruppe mit ein bis neun Kohlenstoffatomen ringsubstituiertem Phenol gebildeten Novolaken.

Die neuen Zusammensetzungen können ferner geeignete Beschleuniger wie tertiäre Amine und quartäre Ammoniumsalze, insbesondere Imidazol, Dimethylaminoäthanol, Pyridin und Tetrabutylammoniumjodid, oder Metalloctanoate, insbesondere Zinkoctanoat oder Stannooctanoat, enthalten. Ferner können Weichmacher wie Dibutylphthalat und Dioctylphthalat, inerte Verdünnungsmittel wie Teere und Bitumen und sogenannte reaktionsfähige Verdünnungsmittel, insbesondere Monoepoxide wie n-Butyl-glycidyläther, Isooctylglycidyläther, Phenyl-glycidyläther, Kresyl-glycidyläther, die Glycidylester von tertiären aliphatischen Monocarbonsäuren, Acrylsäureglycidylester und Methacrylsäureglycidylester, in den Zusammensetzungen vorliegen. Ferner können sie Zusatzstoffe, wie Füllstoffe, Verstärkungsmittel, Farbstoffe, Verlaufmittel, Flammhemmstoffe und Formtrennöle enthalten. Als Streckmittel, Füllstoffe und Verstärkungsmittel eignen sich beispielsweise Glasfasern, Kohlenstoffasern, Glaskügelchen, Glimmer, Quarzmehl, Calciumcarbonat, Cellulose, Kaolin, Wollastonit, kolloidale Kieselsäure mit grosser spezifischer Oberfläche, gepulvertes Polyvinylchlorid und gepulverte Polyolefinkohlenwasserstoffe wie Polyäthylen und Polypropylen.

Die erfindungsgemässen härtbaren Zusammensetzungen finden ihren Einsatz als Laminierharze, Sinterpulver, Tränk- und Giessharze, Pressmassen, Kitte und Dichtungsmassen, Einbettungs- und Isoliermassen für die elektrische Industrie und als Klebstoffe sowie in der Herstellung solcher Produkte, und sie sind besonders geeignet zur Verwendung als Überzüge, sowohl als Anstrichmittel und Lacke als auch als Pulverbeschichtungen.

Die nachfolgenden Beispiele erläutern die Erfindung. Teile sind Gewichtsteile, falls nicht anders angegeben.

Als Epoxidharz I wird ein Diglycidyläther des Bisphenols A bezeichnet, der mit Bisphenol A auf einen Epoxidgehalt von 1,3 val/kg vorverlängert ist.

Als Epoxidharz II wird ein Diglycidyläther des Bisphenols A bezeichnet, der einen Epoxidgehalt von 5,23 val/kg aufweist.

Als Epoxidharz III wird ein Diglycidyläther des Bisphenols A bezeichnet, der einen Epoxidgehalt von 5,1 val/kg aufweist.

Als Epoxidharz IV wird N,N'-Diglycidyl-5,5-dimethylhydantoin bezeichnet.

Als Epoxidharz V wird ein dem Epoxidharz I ähnlicher Diglycidyläther bezeichnet, der jedoch auf einen Epoxidgehalt von 1,0 val/kg vorverlängert ist.

Beispiel 1

3-Mercaptopropionsäure (99% rein, 322 g; 3 Mol) wird in einen mit Rührer, Thermometer und Rückflusskühler ausgerüsteten 1 Liter-Glaskolben eingewogen. Den Kolben erhitzt man, bis der Inhalt 95°C erreicht, und gibt dann Melamin (63 g; 0,5 Mol) dazu. Man stellt auf eine Temperatur von 90°C ein, bei welcher der Inhalt flüssig ist, aber noch etwas nicht aufgelöstes Melamin enthält. Im Verlauf von 4 Minuten gibt man eine mit 6% Methanol stabilisierte wässrige 36,5 gew.%ige Formaldehydlösung (246 g; 3 Mol) dazu. Dabei klärt sich das Gemisch, und eine exotherme Reaktion setzt ein. Man hält die Temperatur auf 100°C und kühlt danach das Gemisch zu einer weissen Paste ab.

Die Paste wird mit dem gleichen Gewicht Wasser und dem gleichen Gewicht Äthanol vermischt. Man erhitzt auf 56°C, was eine klare Lösung ergibt. Beim Abkühlen scheiden sich Kristalle aus dieser Lösung ab. Nach Stehen über Nacht werden die Kristalle abfiltriert und aus 50%igem wässrigen Äthanol umkristallisiert. Die Kristalle werden bei 70°C im Vakuumtrockenschrank getrocknet, wobei man Hexakis-(N-2--carboxyäthylthiomethyl)-melamin vom Schmelzpunkt 153-154°C erhält.

Die Elementaranalyse zeigt die folgenden Ergebnisse:

| Berechnet für $C_{27}H_{42}N_6O_{12}S_6$ | | Gefunden |
|---|---|---|
| C | 38,84% | 38,6% |
| H | 5,07% | 5,0% |
| N | 10,06% | 10,1% |
| O | 22,99% | 23,0% |
| S | 23,04% | 23,0% |

Die gefundene Säurezahl dieses Materials beträgt 7,38 val/kg gegenüber einem theoretischen Wert von 7,19 val/kg.

Beispiel 2

Man wiederholt Beispiel 1, jedoch wird am Ende der Reaktion flüchtiges Material durch Destillation entfernt, zunächst bei Atmosphärendruck und dann im Vakuum, wobei das Gemisch zuletzt bis auf 140°C/64 Torr erhitzt wird. Man kühlt das Produkt auf 110°C ab, giesst in eine

flache Schale und lässt die klare farblose viskose Flüssigkeit abkühlen. Das Produkt, rohes Hexakis-(N-2-carboxyäthylthiomethyl)-melamin (421 g; 100% Ausbeute) wird 11 Tage bei 25°-35°C gelagert und wandelt sich dabei in eine harte, weisse, durchscheinende Masse um, die sich zu Pulver zermahlen lässt. Das Pulver weist eine Säurezahl von 7,34 val/kg (theoretischer Wert 7,19 val/kg) auf und schmilzt bei 134-138°C. Eine Probe wird aus 50%igem wässrigen Äthanol umkristallisiert und schmilzt dann bei 148-152°C.

Beispiel 3

Man gibt flüssiges, methyliertes Melamin/ Formaldehydharz (287,5 g; Gehalt an Nichtflüchtigem 97%; Melamin: Formaldehyd: Methyl - Molverhältnis = 1:6:4,5) bei 100°C zu Thioglykolsäure (332,2 g; 98,9% rein). Die Zugabe erfordert 30 Minuten, wobei man die Temperatur auf 90-110°C hält. Dann wird auf 120°C erhitzt, um flüchtige Stoffe abzudestillieren, und die Destillation wird bei 128°C im Wasserstrahlpumpenvakuum zu Ende geführt. Das Destillat wiegt 109 g und besteht aus Methanol aus der Umsetzung zwischen Thioglykolsäure und dem methylierten Harz, flüchtigem Material aus dem methylierten Harz selbst sowie Wasser, Methanol und Dimethoxymethan aus der Kondensation von zwei oder mehr Melaminringen miteinander.

Den noch heissen Destillationsrückstand giesst man auf eine flache Horde und lässt ihn abkühlen. Dieses Material ist ein klarer, harter, farbloser, harziger Feststoff, der bei 62°C erweicht. Gelpermeationschromatographie bestätigt, dass das Molekulargewicht zufolge der Reaktion erheblich angestiegen ist; während das Ausgangsmaterial sich vorwiegend aus einkernigen Typen zusammensetzt, besitzt das Produkt grossenteils ein Molekulargewicht von nahezu 2000, was sich durch Überschreitung der Ausschlussgrenze der Säule zeigt und damit weitere Beweise dafür erbringt, dass zwei oder mehr Melaminringe verknüpft worden sind.

Die Säurezahl dieses Materials liegt bei 4,73 val/kg; der auf die Einsatzmenge Thioglykolsäure berechnete Wert beträgt 7,0 val/kg. Es ist anzunehmen, dass die niedrige Säurezahl eine Folge einer teilweisen Weiterreaktion der Carboxylgruppen im verätherten Produkt mit dem freigesetzten Methanol zu den entsprechenden Methylestern ist. Diese Annahme wird durch Verseifung des Produkts mit Normalnatronlauge bewiesen, wobei die Neutralisationszahl stark zunimmt. Es finden sich keine Anzeichen für eine Veresterung von freigesetztem Methanol mit noch nicht umgesetzter Thioglykolsäure.

Weitere Anzeichen, welche die Bildung der Ester bestätigen, finden sich bei der Untersuchung des $^{13}$C-NMR-Spektrums, wo sich zwei neue Peaks zeigen, keiner von welchen in den Spektren des Melaminharzausgangsmaterials, der Thioglykolsäure oder des Produkts aus Beispiel 7 (siehe unten) vorhanden ist:

Dem Carbonylkohlenstoff zugehörige $^{13}$C-NMR-Verschiebungen (ppm)

| | $\underline{C}OOH$ | $\underline{C}OOCH_3$ |
|---|---|---|
| Thioglykolsäure | 172,0 | keine |
| Produkt aus Beispiel 7 | 171,41 | keine |
| Produkt aus Beispiel 3 | 171,48 | 170,61 |

Dem Methylkohlenstoff zugehörige $^{13}$C-NMR-Verschiebungen (ppm)

| | $CH_2O\underline{C}H_3$ | $COO\underline{C}H_3$ |
|---|---|---|
| Melaminharzausgangsmaterial | 55,02 | keine |
| Produkt aus Beispiel 3 | keine | 52,2 |

Die Verschiebungen der neuen, der Gegenwart von Methylester zuzuschreibenden Peaks sind unterstrichen.

Beispiel 4

«Cymel» 1156 (74,1 g, 0,14 Mol; ein von der American Cyanamid Corporation geliefertes, hoch n-butyliertes Hexamethylolmelamin mit einer Gardner-Holdt Viskosität von $Z_2$-$Z_4$ bei 25°C, einem Stickstoffgehalt von 16,7% und einem Feststoffgehalt von 98%; «Cymel» ist ein Warenzeichen) und 3-Mercaptopropionsäure (45,9 g, 0,42 Mol) werden in einem Erlenmeyerkolben auf einer mit Magnetrührer ausgerüsteten Heizplatte erhitzt. Das Gemisch wird 40 Minuten auf 70°C und danach 5 Minuten auf 110°C erhitzt. Die Thiolzahl des Produkts beträgt weniger als 0,01 Mol/kg, was einem über 99,7%igen Umsatz des urpsrünglich vorhandenen Thiolgruppengehalts entspricht. Die Säurezahl dieses Produkts liegt bei 3,21 val/kg; der berechnete Wert für in dem freigesetzten Reaktions-n-butanol gelöstes Tris-(N-2-carboxyäthylthiomethyl)-tris-(N-butoxymethyl)-melamin beträgt 3,60 val/kg. Die niedrige gefundene Säurezahl wird durch teilweise Veresterung verursacht.

Beispiel 5

Man verfährt wiederum wie in Beispiel 4, unter Verwendung von 78,1 g (0,15 Mol) «Cymel» 1156 und 41,9 g (0,44 Mol) frisch destillierter Thioglykolsäure. Die Thiolzahl des Produkts weist auf über 99%igen Umsatz, und das Produkt hat eine Säurezahl von 3,01 val/kg; der berechnete Wert für im freigesetzten Reaktions-n--butanol gelöstes Tris-(N-carboxymethylthiomethyl)-tris-(N-n-butoxymethyl)-melamin beträgt 3,80 val/kg. Die niedrige gefundene Säurezahl wird durch teilweise Veresterung verursacht.

Beispiel 6

Man erhitzt «Cymel» 1156 (76,0 g, 0,145 Mol) und Thioäpfelsäure (44,0 g, 0,29 Mol) unter Rühren 40 Minuten auf 75-85°C, nach welcher Zeit die Thiolzahl auf über 99%igen Umsatz weist. Man versetzt mit Xylol (23,4 g), was eine klare, viskose, schwach hellbraune Lösung ergibt. Die Säurezahl des Produkts liegt bei 3,68 val/kg (ausgedrückt als der Wert vor der Verdünnung mit Xylol); der berechnete Wert für im freigesetzten

Reaktions-n-butanol gelöstes Bis-(N-1,2-(dicarboxy)-äthylthiomethyl)-tetrakis-(N-n-butoxymethyl)-melamin beträgt 4,09 val/kg. Die niedrige gefundene Säurezahl wird durch teilweise Veresterung verursacht.

Beispiel 7

Man vermischt Thioglykolsäure (282 g; 97,9% rein; 3 Mol) mit Melamin (64 g; 0,5 Mol) und Wasser (200 ml) zu einer einheitlichen viskosen Aufschlämmung. Man versetzt mit Formaldehydlösung (36,3% in Wasser, 246 g; 3 Mol) und erhitzt auf 65°C, wobei sich das Gemisch klärt. Anschliessend erhitzt man 30 Minuten am Rückfluss und destilliert bei Atmosphärendruck. Nach Auffangen von 323 g Destillat und nachdem der Kolbeninhalt 110°C erreicht hat, wird die Destillation unterbrochen und der Rückstand abgekühlt, was 441 g einer klaren viskosen Flüssigkeit liefert. Durch 1stündiges Erhitzen von 1 g auf 120°C findet man einen Gehalt von 87,0% Nichtflüchtigem in diesem Produkt. Dessen auf den Gehalt an Nichtflüchtigem bezogene Säurezahl liegt bei 7,68 val/kg. Der berechnete Wert für Hexakis-(N-carboxymethylthiomethyl)-melamin beträgt 7,99 val/kg.

Beispiel 8

Melamin (32 g; 0,25 Mol), 3-Mercaptopropionsäure (98,5% rein; 80,7 g; 0,75 Mol) und destilliertes Wasser (150 ml) werden zusammen erhitzt und verrührt. Bei 93°C bildet sich eine klare Lösung des Melaminsalzes der 3-Mercaptopropionsäure. Dazu gibt man langsam bei 100°C 36,6%ige wässrige Formaldehydlösung (61,5 g; 0,75 Mol). Die Zugabe erfordert 7 Minuten, wobei wegen der exothermen Natur der Reaktion die Temperatur ohne äussere Wärmezufuhr bei 85-90°C bleibt. Die Lösung trübt sich, und nach weiteren 6 Minuten Rühren ist kein Formaldehydgeruch mehr nachweisbar. Danach stellt man den Rührer ab und lässt das Gemisch abkühlen. Die obere Schicht wird dann von einer weisslichen klebrigen unteren Schicht abgegossen, welche man weiter abkühlt, zerdrückt, abfiltriert und mit eiskaltem Wasser wäscht. Der Feststoff wird im Vakuumtrockenschrank bei 40°C getrocknet, wobei man 114 g (93,7% der Theorie) symmetrisches und/oder asymmetrisches Tris-(N-2-carboxyäthylthiomethyl)-melamin als weisses Pulver mit einem Schmelzpunkt von 166° bis 175°C und einer Säurezahl von 6,01 val/kg (theoretischer Wert 6,24 val/kg) erhält. Das Produkt besitzt keinen nachweisbaren Thiolgehalt.

Beispiel 9

Acetoguanamin (6,25 g; 0,05 Mol) und 3-Mercaptopropionsäure (21,23 g; 0,20 Mol) werden zusammen verrührt und erhitzt. Wenn 85°C erreicht sind, klärt sich das Gemisch, und bei 95°C versetzt man mit 36,0%igem wässrigen Formaldehyd (16,67 g; 0,20 Mol) und danach mit destilliertem Wasser (20 ml). Man erhitzt auf 100°C, und dabei bildet sich ein weisser Niederschlag. Nach weiteren 20 Minuten Erhitzen bei 100°C kühlt man das Gemisch auf Raumtemperatur ab, filtriert und wäscht den Niederschlag in kaltem Wasser. Nach Trocknung im Vakuumtrockenschrank beträgt die Ausbeute 18,7 g (62,6% der Theorie); das Produkt schmilzt bei 220-227°C. Dessen Säurezahl liegt bei 6,64 val/kg, während der theoretische Wert für Tetrakis--(N-2-carboxyäthylthiomethyl)-acetoguanamin 6,69 val/kg beträgt.

Beispiel 10

Benzoguanamin (9,35 g; 0,05 Mol) und 3-Mercaptopropionsäure (21,23 g; 0,20 Mol) werden zusammen verrührt und erhitzt. Bei 96°C klärt sich das Gemisch, und man versetzt mit 36,0%-igem wässrigen Formaldehyd (16,67 g; 0,20 Mol). Man hält die Temperatur 20 Minuten bei 82-89°C und bringt dann Kühlung zur Einwirkung. Ein Öl scheidet sich ab, das bei weiterem Rühren erstarrt. Destilliertes Wasser (100 ml) wird zugesetzt und der weisse Feststoff abfiltriert und im Vakuumtrockenschrank getrocknet. Die Ausbeute an rohem Material beträgt 28,8 g (87,3% der Theorie); dieses schmilzt bei 110-118°C. Die Säurezahl des Materials liegt bei 6,30 val/kg, während der theoretische Wert für Tetrakis-(N-2-carboxyäthylthiomethyl)-benzoguanamin 6,06 val/kg beträgt.

Beispiel 11

Man vermischt «Cymel» 1156 (38,8 g), 2-Äthylhexylthioglykolat (40,8 g) und 3-Mercaptopropionsäure (21,2 g) in einem Kolben. Das Molverhältnis dieser Komponenten beträgt 1:2,63:2,63. Man verbindet den Kolben mit einem Rotationsverdampfer und erhitzt 9½ Stunden im Wasserbad bei 95°C. Ein Vakuum wird angelegt, und im Verlauf von 2 Stunden sammeln sich 16,9 g Destillat. Das im Kolben verbliebene Produkt (82,6 g) besitzt eine Thiozahl von 0,22 val/kg, was auf über 95%igen Umsatz weist.

Es handelt sich um eine farblose, leicht giessbare, schwachtrübe Flüssigkeit mit einer Säurezahl von 2,35 val/kg. Die berechnete Säurezahl für ein Tris-(N-2-carboxyäthylthiomethyl)-tris-N--2-(äthyl)-hexyloxycarbonylmethylthiomethyl)-melamin beträgt 2,66 val/kg.

Beispiel 12

Man erhitzt Benzoguanamin (9,35 g; 0,05 Mol), 3-Mercaptopropionsäure (21,23 g; 0,20 Mol) und n-Butyraldehyd (14,42 g; 0,20 Mol) unter Rühren auf 64°C, bei welcher Temperatur die Reaktion exotherm wird. Nach 30 Minuten bei 50-60°C ist das Gemisch eine klare Flüssigkeit mit Spuren eines suspendierten Feststoffs. Man kühlt auf Raumtemperatur ab, was eine viskose breiige Masse ergibt.

Das Produkt wird in kaltem Aceton (50 ml) suspendiert und filtriert. Den Rückstand wäscht man zweimal mit 25 ml kaltem Aceton und trocknet, was 13,7 g weisses Pulver vom Schmelzpunkt 142-148°C ergibt. Dieses Produkt weist eine Säurezahl von 4,1 val/kg auf, was anzeigt,

dass durchschnittlich 3,4 an Stickstoff im Benzoguanaminmolekül gebundene Wasserstoffatome durch Gruppen der Formel

$$\begin{array}{l} CH_2CH_2CH_3 \\ | \\ -CHSCH_2CH_2COOH \end{array} \qquad XIX$$

ersetzt worden sind.

Beispiel 13

Man erhitzt Melamin (31,6 g; 0,25 Mol), 3-Mercaptopropionsäure (79,6 g; 0,75 Mol) und destilliertes Wasser (150 g); wenn das Gemisch 83°C erreicht, bildet sich eine klare Flüssigkeit. Diese wird dann auf 75°C abgekühlt und langsam mit n-Butyraldehyd (54,0 g; 0,75 Mol) versetzt, wobei man die Temperatur zwischen 70 und 80°C hält.

Nach beendeter Zugabe erhitzt man 4½ Stunden am Rückfluss.

Das Gemisch wird abgekühlt und die klare wässrige Schicht entfernt. Man wäscht die organische Schicht mit destilliertem Wasser (2×250 ml) und löst sie dann in 10%iger Natriumcarbonatlösung (500 ml). Die Lösung wird mit Essigsäurelösung (10%) neutralisiert, wodurch das Produkt ausgefällt wird. Dieses wird abfiltriert und getrocknet, was 106,5 g symmetrisches und/oder asymmetrisches Tris-(N-1-(2-carboxyäthylthio)-butyl)-melamin als weissen Feststoff mit einer Säurezahl von 4,40 val/kg ergibt. Die berechnete Säurezahl für Tris-(N-1-(2-carboxyäthylthio)-butyl)-melamin beträgt 4,54 val/kg. Das $^{13}C$-NMR-Spektrum des Produkts steht mit einer solchen Verbindung im Einklang.

Beispiel 14

Man erhitzt Lauroguanamin (53,1 g, 0,2 Mol), 3-Mercaptopropionsäure (84,9 g, 0,8 Mol) und 50 g Wasser auf 85°C, wobei das Gemisch zwei flüssige Phasen bildet. Dann versetzt man tropfenweise mit 65,8 g (0,8 Mol) 36,5%iger wässriger Formaldehydlösung. Nach Zugabe von etwa 15 g wird das Reaktionsgemisch eine klare gelbe Lösung. Die Zugabe des Formaldehyds wird im Verlauf von vier Minuten zu Ende geführt, wobei die Temperatur des Reaktionsgemischs auf 93°C steigt. Man erhitzt dann 30 Minuten am Rückfluss (98°-100°C), währenddessen ein weisser Feststoff ausfällt. Nach Abkühlen wird das Gemisch filtriert und der weisse Feststoff mehrmals mit heissem Wasser gewaschen. Die Produktausbeute beträgt 109 g nach Trocknung im Vakuum. Das Produkt, im wesentlichen Tetrakis-(N-2-carboxyäthylthiomethyl)-lauroguanamin besitzt eine Säurezahl von 4,38 val/kg und eine vernachlässigbar kleine Thiolzahl und schmilzt bei 155°C (auf der Koflerbank bestimmt).

Beispiel 15

Man erhitzt gemäss Beispiel 2 hergestelltes, umkristallisiertes Hexakis-(N-2-carboxyäthylthiomethyl)-melamin (8,34 g, 0,01 Mol) und 2,95 g (0,025 Mol) 2-n-Butoxyäthanol auf 150°C. Anfänglich bildet sich eine dicke, weisse, kaum rührbare Paste, die sich aber nach 40 Minuten in eine dünne, klare, farblose Lösung umwandelt. Man erhitzt noch weitere 100 Minuten auf 150°C und kühlt dann ab. Das Produkt, eine klare farblose Flüssigkeit mit süsslichem Geruch, besitzt eine Säurezahl von 3,48 val/kg (für das anfängliche Gemisch berechneter Wert 5,49 val/kg; der berechnete Wert für ein Produkt, das durchschnittlich 2,5 2-(n-Butoxyäthoxycarbonyl)-äthylthiomethylgruppen pro Molekül enthält, beträgt 3,80 val/kg) und einen Feststoffgehalt (bestimmt durch 60 Minuten Erhitzen einer Probe von 0,5 g auf 120°C) von 92,4% (berechneter Anfangswert 73,9%).

Beispiel 16

Man bereitet eine Zusammensetzung mit folgendem Gehalt:

| | | |
|---|---|---|
| 60%ige | Lösung von Epoxidharz I in EEA[a] | 200 g |
| 50%ige | Lösung des Produkts aus Beispiel 2 in EEA | 44 g |
| 10%ige | Lösung von Polyacrylsäurebutylester[b] in EEA | 7,5 g |

[a] EEA bezeichnet 2-Äthoxyäthylacetat
[b] als Verlaufmittel zugesetzt.

Man gibt eine weitere Menge 2-Äthoxyäthylacetat dazu, um die Viskosität des Gemischs auf etwa 80 mPa s zu verringern.

Stahlplatten werden mit dieser Lösung gespritzt und 15 Minuten bei 200°C eingebrannt.

An den Überzügen werden die folgenden Beobachtungen und Prüfungen vorgenommen:

TABELLE I

| | |
|---|---|
| Aussehen | klare glatte gelbe Filme |
| Stärke | 15-25 μ |
| Widerstand gegen Aceton (1 Minute Berührung mit einem in Aceton getränkten Wattebausch) | sehr geringer Effekt |
| Schlagfestigkeit auf der Rückseite[c] | positiv |
| Dehnbarkeit (DIN 53156) | 9,5 mm |

[c] Bei der Schlagfestigkeitsprüfung auf der Rückseite lässt man ein zugespitztes 2 kg-Gewicht, bei dem der grösste Durchmesser der Aufschlagfläche 2 cm beträgt, aus einer Höhe von 80 cm auf die Rückseite der beschichteten Platte fallen. Ein «positives» Ergebnis bedeutet, dass der Überzug dabei nicht brach.

Diese Ergebnisse zeigen, dass die Überzüge vernetzt sind und sehr gute mechanische Eigenschaften aufweisen.

Beispiel 17

Anteile der Produkte aus Beispielen 4, 5 und 6 werden jeweils getrennt mit der stöchiometrischen Menge Epoxidharz II vermischt und mit 2-n-Butoxyäthanol verdünnt. Die Formulierungen sind in der nachfolgenden Tabelle angegeben:

TABELLE II

| | Zusammensetzung (Teile) A | B | C |
|---|---|---|---|
| Produkt aus Beispiel 4 | 244 | — | — |
| Produkt aus Beispiel 5 | — | 261 | — |
| Produkt aus Beispiel 6 | — | — | 213 |
| Epoxidharz II | 150 | 150 | 150 |
| 2-n-Butoxyäthanol | 40 | 30 | 80 |

Ungefähr 100 μ starke Filme aus diesen Zusammensetzungen werden auf Glasplatten gegossen, 1 Stunde bei Raumtemperatur getrocknet und dann wie unten angegeben ½ Stunde eingebrannt. Die Ergebnisse sind in der nachfolgenden Tabelle angeführt:

TABELLE III

| Zusammensetzung | Härtungstemperatur | Aussehen | Widerstand gegen AMK[d] | Härte[e] |
|---|---|---|---|---|
| A | 120°C | Klar, farblos, glänzend | Unbeeinflusst | Unbeeinflusst |
| B | 120°C | Klar, farblos, glänzend | Unbeeinflusst | Unbeeinflusst |
| C | 140°C | Klar, schwachgelb, glänzend | Unbeeinflusst | Unbeeinflusst |

[d] Der Widerstand gegen AMK wird bestimmt, indem man den Überzug 20mal mit einem in Äthylmethylketon getränkten Wattebausch hin hin und her reibt.

[e] Die Härte wird beurteilt, indem man den Film mit dem Fingernagel anzukratzen versucht.

Beispiel 18

Man vermischt das Produkt aus Beispiel 7 (5,0 g) mit Epoxidharz III (6,8 g) und verdünnt mit 2-Äthoxyäthanol (5 ml), was eine klare Lösung ergibt. Ein Film dieser Lösung wird auf eine Glasplatte gegossen und 30 Minuten bei 100°C eingebrannt, was einen zähen, harten Film ergibt, der bei 10maligem Reiben mit einem in Aceton getränkten Wattebausch unbeeinflusst bleibt.

Beispiel 19

Proben von 1,0 g Gewicht der Produkte aus Beispielen 8, 9 und 10 werden in Dimethylaminoäthanol (DMAE) und Wasser bzw. in Dimethylformamid (DMF) gelöst und mit dem oben beschriebenen Epoxidharz IV bzw. Epoxidharz II vermischt. Die Gemische werden zu 100 μ Stärke auf Glasplatten gegossen und 30 Minuten im Ofen bei 150°C eingebrannt. Darauf unterwirft man sie 20maligem Hinundherreiben mit einem in Äthylmethylketon getränkten Wattebausch. Die Ergebnisse sind wie in der folgenden Tabelle angeführt:

TABELLE IV

| Produkt aus Beispiel | DMAE (g) | Wasser (g) | DMF (g) | Epoxidharz Typ | Menge (g) | Aussehen nach dem Einbrennen | Widerstand gegen AMK |
|---|---|---|---|---|---|---|---|
| 8 | 0,65 | 1,27 | — | IV | 2,92 | hart, orange-gelb | geringes Erweichen |
| 9 | 0,76 | 1,19 | — | IV | 2,95 | hart, gelb-braun | unbeeinflusst |
| 10 | — | — | 2,19 | II | 1,19 | hart, klar, glänzend | unbeeinflusst |

Beispiel 20

Man vermischt das Produkt aus Beispiel 11 (1 g) mit 4,3 g einer 60%igen Lösung des Epoxidharzes V in 2-n-Butoxyäthanol und verdünnt mit 2-Äthoxyäthanol (1 g).

Ein Film von 75 µ Nassdicke wird auf eine Glasplatte gegossen und dann 30 Minuten auf 150°C erhitzt. Danach erweist sich der Film als klar und zäh. Bei 20maligem Reiben mit in Xylol getränkter Watte bleibt er unbeeinflusst und erleidet nur eine geringe Erweichung, wenn er 20mal mit in 2-Äthoxyäthanol getränkter Watte gerieben wird.

Beispiel 21

Der in Beispiel 15 hergestellte Teilester (1 g) wird mit 5,87 g einer 60%igen Lösung des Epoxidharzes V in 2-n-Butoxyäthanol vermischt und ineiner 75 µ dicken Schicht auf eine Glasplatte gestrichen. Die Schicht wird 30 Minuten bei 150°C gehärtet, wobei sich ein klarer, farbloser, glänzender, zäher, biegsamer Film bildet, der bei 20maligem Reiben mit einem in 2-n-Butoxyäthanol getränkten Wattebausch unbeeinflusst bleibt.

In einem weiteren Versuch erhitzt man ein Gemisch von 5,24 g des Produkts aus Beispiel 15 und 3,48 g Epoxidharz II 30 Minuten auf die niedrigere Temperatur von 100°C. Es entsteht ein Film mit ähnlichen Eigenschaften.

**Ansprüche**

1. Aminotriazinpolycarbonsäuren und deren Teilester mit mindestens zwei freien Carboxylgruppen, dadurch gekennzeichnet, dass sie der allgemeinen Formel

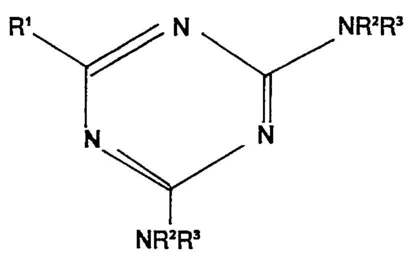

I

worin $R^1$ für eine Gruppe der Formel $-NR^2R^3$, eine geradkettige oder verzweigte Alkylgruppe mit 1 bis 17 Kohlenstoffatomen, eine einkernige Arylgruppe mit 6 bis 10 Kohlenstoffatomen, eine Phenylalkylengruppe mit 1 bis 4 Kohlenstoffatomen im Alkylenteil oder eine Gruppe der Formel

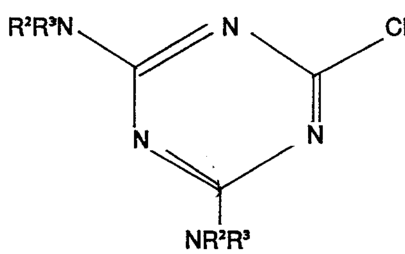

II

steht, $R^2$ und $R^3$ gleich oder verschieden sein können und je ein Wasserstoffatom, eine Gruppe der Formel $-CHR^4OR^5$, eine Gruppe der Formel $-CHR^4-S-R^6-COOQ$ oder eine Gruppe der Formel

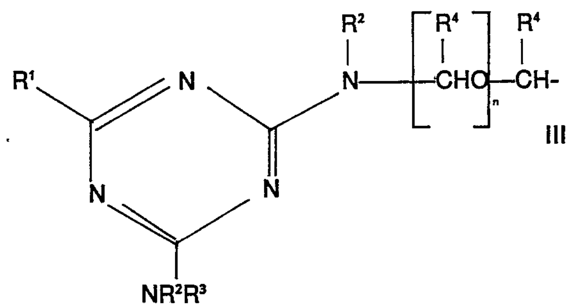

III

darstellen, wobei $R^2$ und $R^3$ in den Formeln I und III so ausgewählt werden, dass höchstens eine davon eine Gruppe der Formel III und mindestens zwei davon eine Gruppe der Formel $-CHR^4-S-R^6-COOQ$ bedeuten, m eine ganze Zahl von 1 bis 3 ist, $R^4$ jeweils für ein Wasserstoffatom oder eine geradkettige oder verzweigte Alkylgruppe mit 1 bis 3 Kohlenstoffatomen, $R^5$ jeweils für ein Wasserstoffatom oder eine geradkettige oder verzweigte Alkylgruppe mit 1 bis 8 Kohlenstoffatom und $R^6$ jeweils für eine gegebenenfalls durch eine weitere Gruppe der Formel -CCOQ substituierte, zweiwertige geradkettige oder verzweigte Alkylengruppe mit 1 bis 4 Kohlenstoffatomen stehen, Q jeweils ein Wasserstoffatom oder eine geradkettige oder verzweigte Alkylgruppe mit 1 bis 8 Kohlenstoffatomen darstellt, derart dass im Durchschnitt jeweils mindestens zwei Q in der Formel I für ein Wasserstoffatom stehen, und n null oder 1 ist, entsprechen.

2. Säuren und Ester nach Anspruch 1, dadurch gekennzeichnet, dass sie ein Molekulargewicht von höchstens 2000 aufweisen.

3. Säuren und Ester nach Anspruch 1 oder 2, dadurch gekennzeichnet, dass alle $R^4$ je ein Wasserstoffatom darstellen.

4. Säuren und Ester nach einem der vorhergehenden Ansprüche, dadurch gekennzeichnet, dass alle $R^6$ jeweils eine Methylen-, Äthylen-, Äthyliden- oder 2-Carboxyäthylidengruppe darstellen.

5. Säuren und Ester nach Anspruch 4, dadurch gekennzeichnet, dass sie ferner im wesentlichen der Formel

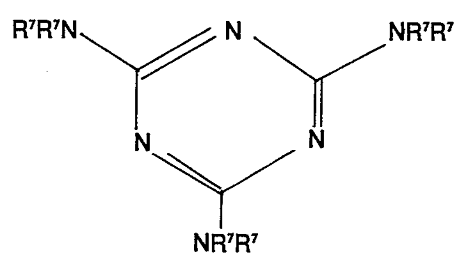

IV

entsprechen, worin zwei bis sechs von den $R^7$ je für eine Gruppe der Formel $-CH_2SCH_2COOQ$, $-CH_2SCH_2CH_2COOQ$, $-CH_2SCH(COOQ)CH_2COOQ$ oder $-CH_2SCH(CH_3)COOQ$ stehen, wobei Q die in Anspruch 1 angegebene Bedeutung hat und

gegebenenfalls verbleibende $R^7$ je ein Wasserstoffatom bedeuten.

6. Säuren und Ester nach Anspruch 4, dadurch gekennzeichnet, dass sie ferner im wesentlichen der Formel

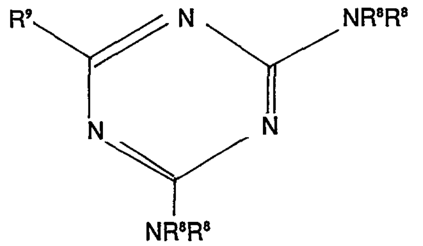

V

entsprechen, worin zwei bis vier von den $R^8$ je für eine Gruppe der Formel $-CH_2SCH_2COOQ$, $-CH_2SCH_2CH_2COOQ$, $-CH_2SCH(COOQ)CH_2COOQ$ oder $-CH_2SCH(CH_3)COOQ$ stehen, wobei Q die in Anspruch 1 angegebene Bedeutung hat und gegebenenfalls verbleibende $R^8$ je ein Wasserstoffatom bedeuten und $R^9$ eine Methyl-, Phenyl- oder Undecylgruppe darstellt.

7. Verfahren zur Herstellung von Aminotriazinpolycarbonsäuren oder deren Teilestern nach einem der vorhergehenden Ansprüche, dadurch gekennzeichnet, dass man mindestens 2 Moläquivalente einer Mercaptocarbonsäure der allgemeinen Formel

$$HS - R^6 - COOH \qquad VI$$

und gegebenenfalls einen gewünschten Anteil eines Esters der Formel

$$HS - R^6 - COOQ^1 \qquad X$$

mit einem Moläquivalent eines gegebenenfalls alkylierten Kondensationsprodukts aus einem Aminotriazin und einem Aldehyd, der allgemeinen Formel

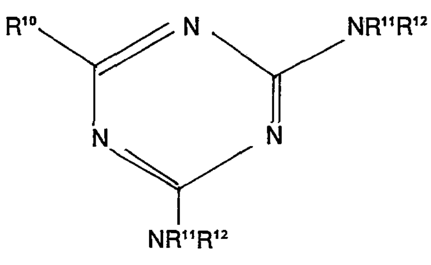

VII

worin $R^6$ die in Anspruch 1 oder 4 angegebene Bedeutung hat, $Q^1$ für eine geradkettige oder verzweigte Alkylgruppe mit 1 bis 8 Kohlenstoffatomen steht, $R^{10}$ eine Gruppe der Formel $-NR^{11}R^{12}$ oder eine geradkettige oder verzweigte Alkylgruppe mit 1 bis 17 Kohlenstoffatomen, eine einkernige Arylgruppe mit 6 bis 10 Kohlenstoffatomen, eine Phenylalkylengruppe mit 1 bis 4 Kohlenstoffatomen im Alkylenteil oder eine Gruppe der Formel

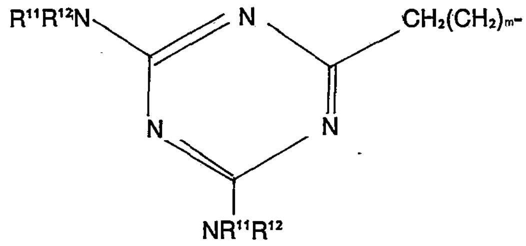

VIII

darstellt, $R^{11}$ und $R^{12}$ gleich oder verschieden sein können und je ein Wasserstoffatom, eine Gruppe der Formel $-CHR^4-O-R^5$ oder eine Gruppe der Formel

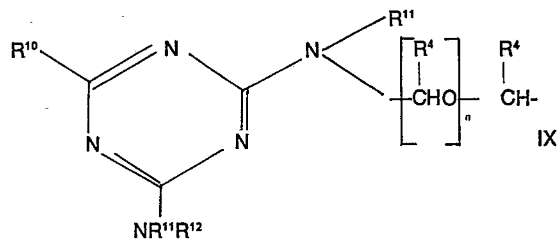

IX

bedeuten und $R^4$ die in Anspruch 1 oder 3 sowie $R^5$, m und n die in Anspruch 1 angegebene Bedeutung haben, wobei $R^{11}$ und $R^{12}$ in der Verbindung der Formel VII so ausgewählt werden, dass höchstens eine davon eine Gruppe der Formel IX und mindestens zwei davon eine Gruppe der Formel $-CHR^4-OR^5$ darstellen, umsetzt.

8. Verfahren zur Herstellung von Aminotriazinpolycarbonsäuren oder deren Teilestern nach einem der Ansprüche 1 bis 6, dadurch gekennzeichnet, dass man mindestens zwei Moläquivalente einer Mercaptocarbonsäure der Formel

$$HS - R^6 - COOH \qquad VI$$

und gegebenenfalls einen gewünschten Anteil eines Esters der Formel

$$HS - R^6 - COOQ^1 \qquad X$$

mit einem Moläquivalent eines Aminotriazins der Formel

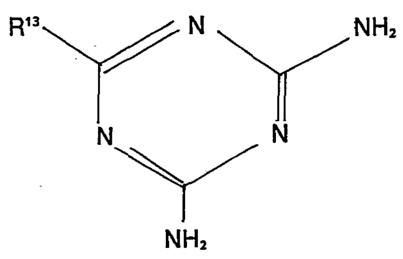

XII

und mindestens zwei Moläquivalenten eines Aldehyds der Formel

$$R^4CHO \qquad XIII$$

worin $R^6$ die in Anspruch 1 oder 4 und $Q^1$ die in Anspruch 7 angegebene Bedeutung haben, $R^{13}$ eine unsubstituierte Aminogruppe, eine geradkettige oder verzweigte Alkylgruppe mit 1 bis 17 Kohlenstoffatomen, eine einkernige Arylgruppe mit 6 bis 10 Kohlenstoffatomen, eine Phenylalkylengruppe mit 1 bis 4 Kohlenstoffatomen im Alkylenteil oder eine Gruppe der Formel

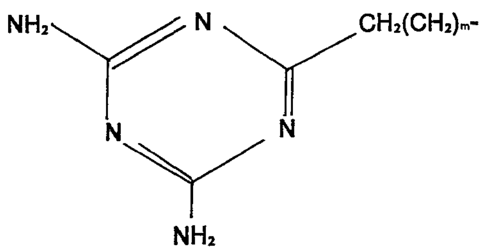

XIV

darstellt sowie m die in Anspruch 1 und $R^4$ die in Anspruch 1 oder 3 angegebene Bedeutung haben, umsetzt.

9. Verfahren zur Herstellung eines Teilesters nach Anspruch 1 bis 6, dadurch gekennzeichnet, dass man eine Aminotriazinpolycarbonsäure nach einem der Ansprüche 1 bis 6, worin mindestens drei Q je ein Wasserstoffatom bedeuten, mit einem Alkohol der Formel $Q^1OH$, worin $Q^1$ die in Anspruch 7 angegebene Bedeutung hat, teilweise verestert.

10. Verwendung von Aminotriazinpolycarbonsäuren oder deren Teilestern nach einem der Ansprüche 1 bis 6 als Härter für Epoxidharze.

**Claims**

1. An aminotriazinepolycarboxylic acid or a partial ester thereof containing at least two free carboxyl groups, characterised by the general formula

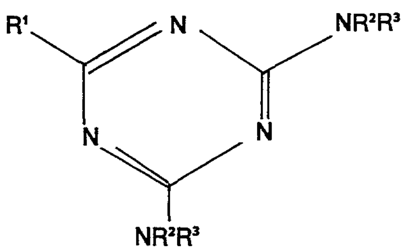

I

wherein

$R^1$ represents a group of formula $-NR^2R^3$, a straight chain or branched alkyl group of 1 to 17 carbon atoms, a mononuclear aryl group of 6 to 10 carbon atoms, a phenylalkylene group containing 1 to 4 carbon atoms in the alkylene moiety, or represents a group of formula

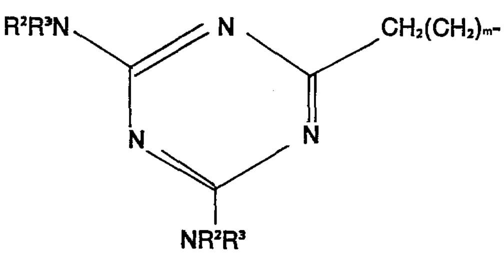

II

$R^2$ and $R^3$ may be the same or different and each represents a hydrogen atom, a group of formula $-CHR^4OR^5$, a group of formula

$$-CHR^4-S-R^6-COOQ$$

or a group of formula

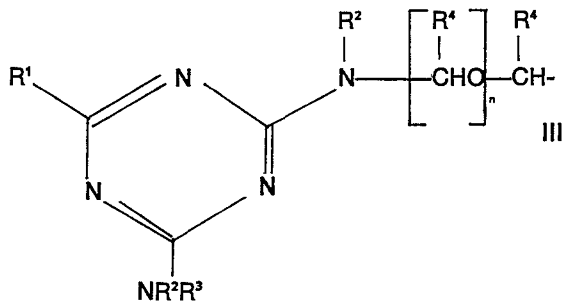

III

$R^2$ and $R^3$ in formulae I and III being selected such that at most one of them represents a group of formula III and at least two of them each represent a group of the formula $-CHR^4-S-R^6-COOQ$, m represents an integer from 1 to 3, each $R^4$ represents a hydrogen atom or a straight chain or branched alkyl group of 1 to 3 carbon atoms, each $R^5$ represents a hydrogen atom or a straight chain or branched alkyl group of 1 to 8 carbon atoms, each $R^6$ represents a divalent straight chain or branched alkylene group of 1 to 4 carbon atoms which is unsubstituted or substituted by a further group of the formula -COOQ, each Q represents a hydrogen atom or a straight chain or branched alkyl group of 1 to 8 carbon atoms, such that on average at least two symbols Q in formula I each represent hydrogen, and n is zero or 1.

2. An acid or ester according to claim 1, having a molecular weight of at most 2000.

3. An acid or ester according to either of claims 1 or 2, wherein each $R^4$ represents a hydrogen atom.

4. An acid or ester according to one of the preceding claims, wherein each $R^6$ represents a methylene, ethylene, ethylidene, or 2-carboxyethylidene group.

5. An acid or ester according to claim 4, which also corresponds substantially to the formula

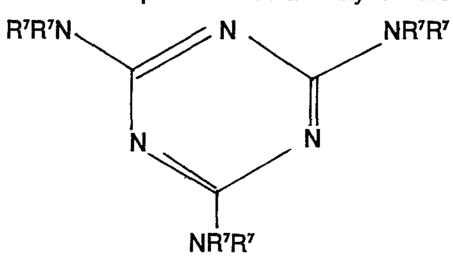

IV

wherein from two to six of $R^7$ each denote a group of formula $-CH_2SCH_2COOQ$, $-CH_2SCH_2CH_2COOQ$, $-CH_2SCH(COOQ)CH_2COOQ$, or $-CH_2SCH(CH_3)COOQ$, where Q is as defined in claim 1, and any remaining $R^7$ each represent a hydrogen atom.

6. An acid or ester according to claim 4, which also corresponds substantially to the formula

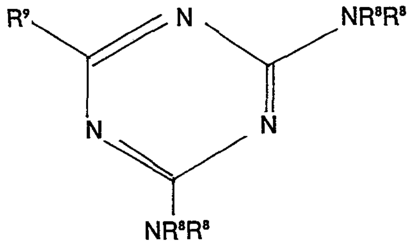

V

wherein from two to four of $R^8$ each denote a group of formula $-CH_2SCH_2COOQ$, $-CH_2SCH_2CH_2-COOQ$, $-CH_2SCH(COOQ)CH_2COOQ$, or $-CH_2SCH(CH_3)COOQ$, wherein Q is as defined in claim 1, and any remaining $R^8$ each represent a hydrogen atom, and $R^9$ represents a methyl, phenyl, or undecyl group.

7. A process for the preparation of an aminotriazinepolycarboxylic acid or a partial ester thereof according to any one of the preceding claims, which process comprises reacting at least 2 molar equivalents of a mercaptocarboxylic acid of the general formula

$$HS - R^6 - COOH \qquad VI$$

and optionally a desired amount of an ester of the formula

$$HS - R^6 - COOQ^1 \qquad X$$

with one molar equivalent of an optionally alkylated condensation product of an aminotriazine and an aldehyde, of the general formula

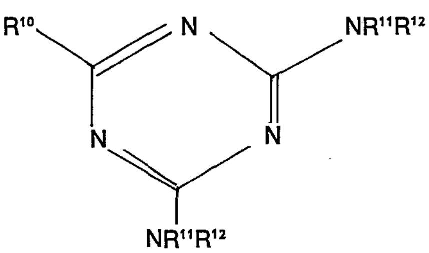

VII

wherein $R^6$ is as defined in claim 1 or claim 4, $Q^1$ is a straight chain or branched alkyl group of 1 to 8 carbon atoms, $R^{10}$ is a group of the formula $-NR^{11}R^{12}$ or a straight chain or branched alkyl group of 1 to 17 carbon atoms, a mononuclear aryl group of 6 to 10 carbon atoms, a phenylalkylene group containing 1 to 4 carbon atoms in the alkylene moiety, or a group of the formula

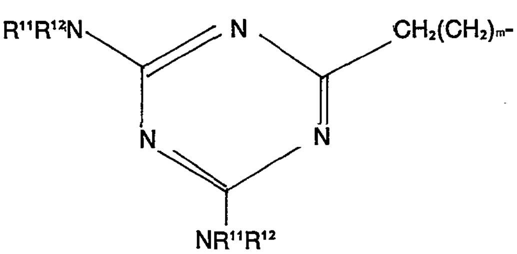

VIII

$R^{11}$ and $R^{12}$ may be the same or different and each is a hydrogen atom, a group of the formula $-CHR^4-O-R^5$ or a group of the formula

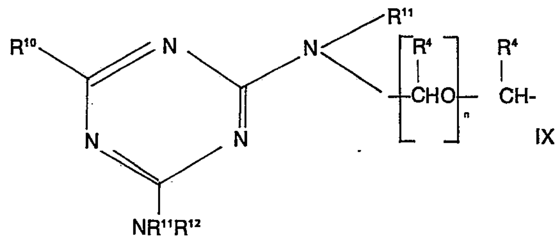

IX

and $R^4$ is as defined in claim 1 or claim 3 and $R^5$, m and n are as defined in claim 1, and $R^{11}$ and $R^{12}$ in the compound of formula VII are chosen such that at most one is a group of the formula IX and at least two are a group of the formula $-CHR^4-OR^5$.

8. A process for the preparation of an aminotriazinepolycarboxylic acid or a partial ester thereof according to any one of claims 1 to 6, which process comprises reacting at least two molar equivalents of a mercaptocarboxylic acid of the formula

$$HS - R^6 - COOH \qquad VI$$

and optionally a desired amount of an ester of the formula

$$HS - R^6 - COOQ^1 \qquad X$$

with one molar equivalent of an aminotriazine of the formula

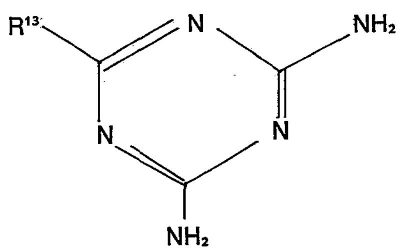

XII

and at least two molar equivalents of an aldehyde of the formula

$$R^4CHO \qquad XIII$$

wherein $R^6$ is as defined in either claim 1 or claim 4 and $Q^1$ is as defined in claim 7, $R^{13}$ is an unsubstituted amino group, a straight chain or branched alkyl group of 1 to 17 carbon atoms, a mononuclear aryl group of 6 to 10 carbon atoms, a phenylalkylene group containing 1 to 4 carbon atoms in the alkylene moiety, or a group of the formula

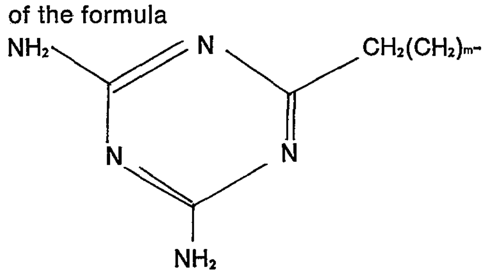

XIV

m is as defined in claim 1 and $R^4$ is as defined in claim 1 or claim 3.

9. A process for the preparation of a partial ester according to any one of claims 1 to 6, which process comprises partially esterifying an aminotriazinepolycarboxylic acid according to any one of claims 1 to 6, wherein at least three of the symbols Q are each a hydrogen atom, with an alcohol of the formula $Q^1OH$, wherein $Q^1$ is as defined in claim 7.

10. Utilisation of an aminotriazinepolycarboxylic acid or a partial ester thereof according to any one of claims 1 to 6 as hardener for epoxy resins.

## Revendications

1. Acides aminotriazinepolycarboxyliques et leurs esters partiels avec au moins deux groupes carboxyles libres, caractérisés en ce qu'ils répondent à la formule générale

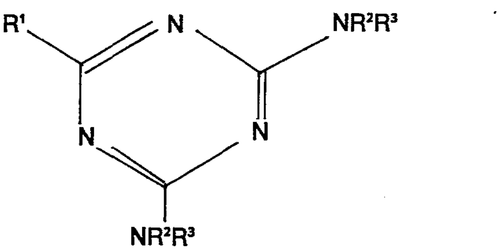

I

dans laquelle $R^1$ désigne un groupe répondant à la formule $-NR^2R^3$, un groupe alkyle linéaire ou ramifié en $C_1$ à $C_{17}$, un groupe aryle mononucléaire en $C_6$ à $C_{10}$, un groupe phénylalkylène ayant 1 à 4 atomes de carbone dans la partie alkylène, ou un groupe répondant à la formule

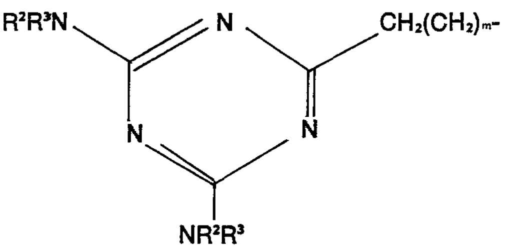

II

$R^2$ et $R^3$ peuvent être indentiques ou différents et désigner chacun un atome d'hydrogène, un groupe répondant à la formule $-CHR^4OR^5$, un groupe répondant à la formule $-CHR^4-S-R^6-COOQ$ ou un groupe répondant à la formule

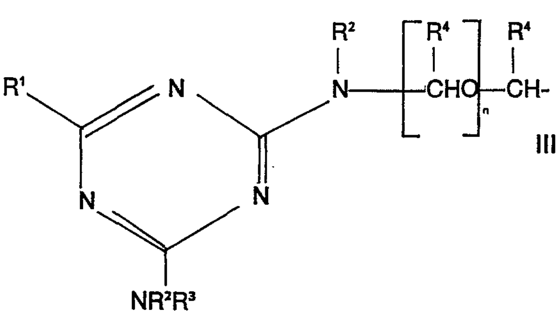

III

les radicaux $R^2$ et $R^3$ des formules I et III étant choisis de telle façon qu'au plus un d'entre eux désigne un groupe répondant à la formule III et au moins deux d'entre eux un groupe répondant à la formule $-CHR^4-S-R^6-COOQ$, m est un nombre entier de 1 à 3, $R^4$ désigne à chaque fois un atome d'hydrogène ou un groupe alkyle linéaire ou ramifié en $C_1$ à $C_3$, $R^5$ désigne à chaque fois un atome d'hydrogène ou un groupe alkyle linéaire ou ramifié en $C_1$ à $C_8$ et de préférence en $C_1$ à $C_4$ et $R^6$ désigne à chaque fois un autre groupe alkylène en $C_1$ à $C_4$ divalent linéaire ou ramifié éventuellement substitué par un autre groupe -COOQ, Q représente à chaque fois un atome d'hydrogène ou un groupe alkyle linéaire ou ramifié en $C_1$ à $C_8$, de telle sorte qu'en moyenne à chaque fois au moins deux Q représentent un atome d'hydrogène dans la formule I, et n est égal à 0 ou 1.

2. Acides et esters suivant la revendication 1, caractérisés en ce qu'ils présentent une masse moléculaire de 2000 au maximum.

3. Acides et esters suivant l'une quelconque des revendications 1 ou 2, caractérisés en ce que tous les $R^4$ représentent chacun un atome d'hydrogène.

4. Acides et esters suivant l'une quelconque des revendications précédentes, caractérisés en ce que tous les $R^6$ représentent à chaque fois un groupe méthylène, éthylène, éthylidène ou 2-carboxyéthylidène.

5. Acides et esters suivant la revendication 4, caractérisés en ce qu'ils répondent en outre essentiellement à la formule

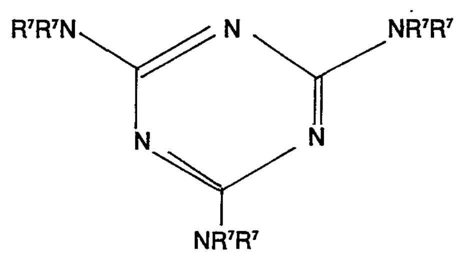

IV

dans laquelle 2 à 6 des $R^7$ désignent chacun un groupe répondant aux formules $-CH_2SCH_2COOQ$, $-CH_2SCH_2CH_2COOQ$, $-CH_2SCH(COOQ)CH_2COOQ$ ou $-CH_2SCH(CH_3)COOQ$, dans lesquelles Q a la signification indiquée dans la revendication 1 et les $R^7$ éventuellement restants désignent chacun un atome d'hydrogène.

6. Acides et esters suivant la revendication 4, caractérisés en ce qu'ils répondent en outre essentiellement à la formule

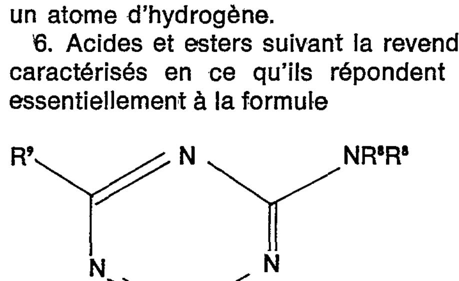

V

dans laquelle deux à quatre des $R^8$ désignent chacun un groupe répondant aux formules $-CH_2SCH_2COOQ$, $-CH_2SCH_2CH_2COOQ$, $-CH_2SCH-$

$(COOQ)CH_2COOQ$ ou $-CH_2SCH(CH_3)COOQ$, Q ayant la signification indiquée dans la revendication 1 et les $R^8$ éventuellement restants désignant chacun un atome d'hydrogène et $R^9$ désignant un groupe métyle, phényle ou undécyle.

7. Procédé de préparation d'acides aminotriazinecarboxyliques ou de leurs esters partiels suivant l'une des revendications précédentes, caractérisé en ce qu'on fait réagir au moins deux équivalents molaires d'un acide mercaptocarboxylique répondant à la formule générale

$$HS - R^6 - COOH \qquad VI$$

et le cas échéant une proportion désirée d'un ester répondant à la formule

$$HS - R^6 - COOQ^1 \qquad X$$

avec un équivalent molaire d'un produit de condensation éventuellement alkylé d'une aminotriazine et d'un aldéhyde, répondant à la formule générale

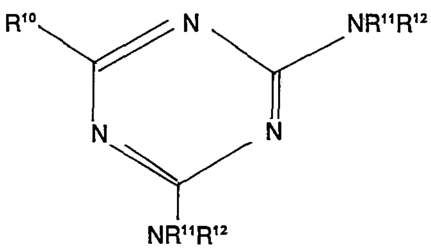

VII

dans laquelle $R^6$ a la signification indiquée dans les revendications 1 ou 4, $Q^1$ désigne un groupe alkyle linéaire ou ramifié en $C_1$ à $C_8$, $R^{10}$ représente un groupe répondant à la formule $-NR^{11}R^{12}$ ou un groupe alkyle linéaire ou ramifié en $C_1$ à $C_{17}$, un groupe aryle mononucléaire en $C_6$ à $C_{10}$, un groupe phénylalkylène ayant 1 à 4 atomes de carbone dans la partie alkylène ou un groupe répondant à la formule

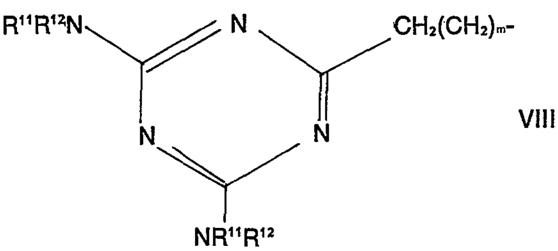

VIII

$R^{11}$ et $R^{12}$ peuvent être identiques ou différents et désignent chacun un atome d'hydrogène, un groupe répondant à la formule $-CHR^4-O-R^5$ ou un groupe répondant à la formule

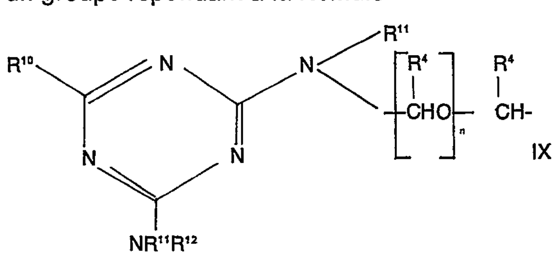

IX

et $R^4$ a la signification indiquée dans les revendications 1 ou 3, $R^5$, m et n ont les significations indiquées dans la revendication 1, $R^{11}$ et $R^{12}$ étant choisis, dans le composé répondant à la formule VII, de telle façon qu'au maximum un d'entre eux représente un groupe répondant à la formule IX et au moins deux d'entre eux un groupe répondant à la formule $-CHR^4-OR^5$.

8. Procédé de préparation d'acides aminotriazine polycarboxyliques ou de leurs esters partiels suivant l'une quelconque des revendications 1 à 6, caractérisé en ce qu'on fait réagir au moins deux équivalents molaires d'un acide mercaptocarboxylique répondant à la formule

$$HS - R^6 - COOH \qquad VI$$

et le cas échéant une proportion désirée d'un ester répondant à la formule

$$HS - R^6 - COOQ^1 \qquad X$$

avec un équivalent molaire d'une aminotriazine répondant à la formule

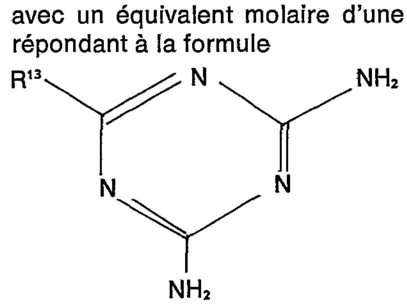

XII

et au moins deux équivalents molaires d'un aldéhyde répondant à la formule

$$R^4CHO \qquad XIII$$

dans laquelle $R^6$ a la signification indiquée dans les revendications 1 ou 4 et $Q^1$ la signification indiquée dans la revendication 7, $R^{13}$ désigne un groupe amino non substitué, un groupe alkyle linéaire ou ramifié en $C_1$ à $C_{17}$, un groupe aryle mononucléaire en $C_6$ à $C_{10}$, un groupe phénylalkylène ayant 1 à 4 atomes de carbone dans la partie alkylène ou un groupe répondant à la formule

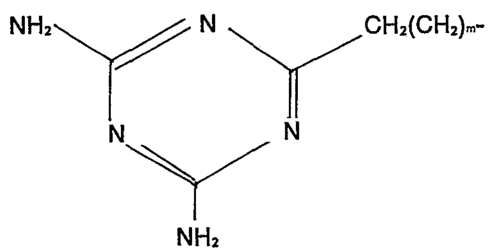

XIV

et m a la signification indiquée dans la revendication 1 et $R^4$ la signification indiquée dans les revendications 1 ou 3.

9. Procédé de préparation d'un ester partiel suivant l'une quelconque des revendications 1 à 6, caractérisé en ce qu'on estérifie partiellement un acide aminotriazine polycarboxylique suivant l'une quelconque des revendications 1 à 6, dans lequel au moins trois Q désignent chacun un atome d'hydrogène, avec un alcool répondant à la formule $Q^1OH$, dans laquelle $Q^1$ a la signification indiquée dans la revendication 7.

10. Utilisation d'acides aminotriazinepolycarboxyliques ou de leurs esters partiels suivant l'une quelconque des revendications 1 à 6 comme durcisseurs pour des résines époxydiques.